# EUROPÄISCHE PATENTSCHRIFT

(11) **EP 2 502 579 B1**
(45) Veröffentlichungstag und Bekanntmachung des Hinweises auf die Patenterteilung: **11.05.2016**
(21) Anmeldenummer: 12160967.1
(22) Anmeldetag: 23.03.2012
(51) Int. Cl.: A61B 17/14

(54) **Chirurgisches Sägeblatt und chirurgische Säge**
Surgical saw blade and surgical saw
Lame de scie chirurgicale et scie chirurgicale

(30) Priorität: 24.03.2011 DE 102011001545
(43) Veröffentlichungstag der Anmeldung: 26.09.2012
(73) Patentinhaber: Aesculap AG, 78532 Tuttlingen (DE)
(72) Erfinder: Högerle, Roland-Alois, 78532 Tuttlingen (DE); Firmbach, Franz-Peter, 78532 Tuttlingen (DE)
(74) Vertreter: Hoeger, Stellrecht & Partner Patentanwälte mbB

(56) Entgegenhaltungen:
- EP-A2- 1 880 682
- DE-U1- 20 012 138
- DE-U1- 20 220 637
- US-A1- 2010 292 701
- US-B2- 7 497 860

## Beschreibung

Die vorliegende Erfindung betrifft ein chirurgisches Sägeblatt mit mindestens einer Zahnreihe und einer Kupplungseinrichtung zum beweglichen Kuppeln und/oder Verbinden des Sägeblatts mit einer chirurgischen, eine Oszillationsachse definierenden Antriebseinheit, wobei das Sägeblatt einen ersten und einen zweiten Sägeblattteil umfasst, wobei der erste Sägeblattteil die mindestens eine Zahnreihe umfasst, wobei der zweite Sägeblattteil die Kupplungseinrichtung umfasst, wobei der erste Sägeblattteil und der zweite Sägeblattteil gelenkig miteinander verbunden sind, wobei der erste Sägeblattteil und der zweite Sägeblattteil relativ zueinander um eine Schwenkachse verschwenkbar aneinander gelagert sind, wobei der zweite Sägeblattteil eine Kupplungsebene definiert und wobei die Kupplungsebene die Schwenkachse enthält.

Ferner betrifft die vorliegende Erfindung eine chirurgische Säge umfassend eine chirurgische Antriebseinheit und ein mit der Antriebseinheit lösbar verbindbares chirurgisches Sägeblatt mit mindestens einer Zahnreihe und einer ersten Kupplungseinrichtung zum beweglichen Kuppeln und/oder Verbinden des Sägeblatts mit der Antriebseinheit, wobei das Sägeblatt einen ersten und einen zweiten Sägeblattteil umfasst, wobei der erste Sägeblattteil die mindestens eine Zahnreihe umfasst, wobei der zweite Sägeblattteil die erste Kupplungseinrichtung umfasst, wobei der erste Sägeblattteil und der zweite Sägeblattteil gelenkig miteinander verbunden sind, wobei der erste Sägeblattteil und dass der zweite Sägeblattteil relativ zueinander um eine Schwenkachse verschwenkbar aneinander gelagert sind, wobei der zweite Sägeblattteil eine Kupplungsebene definiert und wobei die Kupplungsebene die Schwenkachse enthält.

Ein chirurgisches Sägeblatt mit mindestens einer Zahnreihe und einer Kupplungseinrichtung zum beweglichen Kuppeln und/oder Verbinden des Sägeblatts mit einer chirurgischen, eine Oszillationsachse definierenden Antriebseinheit ist beispielsweise aus der DE 102 31 393 A1 bekannt. Derartige Sägeblätter kommen insbesondere bei Operationen zur Präparation von Knochen eines Patienten vor der Implantation einer Endoprothese zum Einsatz. Um Sägeschnitte am Knochen des Patienten in definierter Weise vorzunehmen, werden die Sägeblätter dabei häufig in eine Sägelehre geführt, welche beispielsweise in Form einer Schlitzschablone ausgebildet sein kann. Ein Problem bei solchen Eingriffen ist es, dass es einem Operateur praktisch nicht gelingt, eine eine chirurgische Antriebseinheit und das mit dieser gekoppelte Sägeblatt umfassende chirurgische Säge, die vergleichsweise groß ist, so parallel zur Sägelehre zu führen, dass keine Kippmomente auf die Sägelehre übertragen werden. Da es wünschenswert ist, die Sägelehre möglichst wenig invasiv am Knie zu befestigen, besteht folglich eine durchaus große Gefahr, dass ein Operateur die Sägelehre aus ihrer gewünschten beziehungsweise vorbestimmten Lage bei der Präparation des Knochens verschiebt.

Ein chirurgisches Sägeblatt der eingangs beschriebenen Art ist beispielsweise aus der EP 1 880 682 A2 bekannt. Weitere chirurgische Sägeblätter sind in der US 7,497,860 B2, der US 2010/0292701 A1 sowie der DE 200 12 138 U1 beschrieben.

Es ist daher eine Aufgabe der vorliegenden Erfindung, ein chirurgisches Sägeblatt und eine chirurgische Säge so zu verbessern, dass möglichst keine Kippmomente von der Säge auf die Sägelehre übertragen werden können.

Diese Aufgabe wird bei einem chirurgischen Sägeblatt der eingangs beschriebenen Art erfindungsgemäß dadurch gelöst, dass die Oszillationsachse senkrecht zu der die Schwenkachse enthaltenden Kupplungsebene verläuft.

Durch diese besondere Ausgestaltung des Sägeblatts ist es möglich, vom Operateur auf das Sägeblatt ausgeübte Kippmomente vom ersten Sägeblattteil zu entkoppeln, welcher vorzugsweise in der Sägelehre geführt wird. Dadurch kann es insbesondere vermieden werden, die Sägelehre aus ihrer gewünschten Lage zu verschieben. Der erste Sägeblattteil kann dann ein ideales, Kippmoment entkoppeltes Teil des Sägeblatts in einer von der Sägelehre definierten Ebene bilden und dabei ideal steif sein. Auf diese Weise kann ein Verlust an Schwingwinkel oder Leistung der chirurgischen Säge vermieden werden. In einer Ebene senkrecht zu einem von der Sägelehre definierten Führungsschlitz weist das Sägeblatt insbesondere jedoch einen größtmöglichen Freiheitsgrad auf. Dies wird beim vorgeschlagenen chirurgischen Sägeblatt durch den zweiten Sägeblattteil erreicht, welcher beispielsweise direkt oder indirekt mit der chirurgischen Antriebseinheit verbunden werden kann. Vorteilhaft ist es, dass der erste Sägeblattteil und der zweite Sägeblattteil relativ zueinander verschwenkbar aneinander gelagert sind. Dies gestattet es, die beiden Sägeblattteile relativ zueinander in praktisch einem beliebigen Winkel zu neigen. Mit anderen Worten kann so eine geneigte Krafteinleitung auf eine chirurgische Säge durch einen Operateur ausgeglichen werden, um eine Verschiebung der Sägelehre aus der gewünschten Position zu vermeiden. Günstig ist es, dass der erste Sägeblattteil und der zweite Sägeblattteil relativ zueinander um eine Schwenkachse verschwenkbar aneinander gelagert sind. Insbesondere durch die Wahl der Schwenkachse kann eine Entkopplung zwischen dem ersten Sägeblattteil und eingeleiteten Kippmomenten sicher vermieden werden.

Vorzugsweise umfasst das chirurgische Sägeblatt ein Verbindungsgelenk zum gelenkigen Verbinden des ersten Sägeblattteils und des zweiten Sägeblattteils miteinander. Das Verbindungsgelenk kann zum einen die Funktion zur Herstellung einer Verbindung des ersten und des zweiten Sägeblattteils erfüllen sowie zum anderen ein Gelenk zwischen beiden Sägeblattteilen ausbilden. Dadurch kann ein besonders kompakter Aufbau des chirurgischen Sägeblatts erreicht werden. Dies ist insbesondere wünschenswert, da das Sägeblatt möglichst leicht sein soll, um nach Möglichkeit sehr hohe Dreh- oder Oszillationsfrequenzen zum Sägen zu ermöglichen. Je geringer eine Masse des Sägeblatts ist, umso geringer ist dessen Trägheitsmoment, welches insbesondere bei einem Oszillationsbetrieb eine Oszillationsfrequenz der Säge begrenzt.

Besonders einfach ist der konstruktive Aufwand für die Herstellung des Sägeblatts, wenn das Verbindungsgelenk in Form eines Scharniergelenks ausgebildet ist. Insbesondere können so die beiden Sägeblattteile relativ zueinander verschwenkbar aneinander gelagert werden.

Besonders einfach und kompakt ausbilden lässt sich das Sägeblatt, wenn der erste Sägeblattteil eine Sägeebene definiert und wenn die Sägeebene die Schwenkachse enthält. Dadurch ist es möglich, unerwünscht auf das Sägeblatt wirkende Kippmomente, die über den zweiten Sägeblattteil eingeleitet werden können, vom ersten Sägeblattteil zu entkoppeln.

Günstig ist es ferner, dass der zweite Sägeblattteil eine Kupplungsebene definiert und dass die Kupplungsebene die Schwenkachse enthält.

Diese Ausgestaltung gestattet einen besonders kompakten Aufbau des Sägeblatts und ermöglicht es zudem, beispielsweise über einen chirurgischen Antrieb der chirurgischen Säge auf das Sägeblatt eingeleitete Kippmomente durch die Anordnung der Schwenkachse vom ersten Sägeblattteil abzukoppeln.

Ein Aufbau des chirurgischen Sägeblatts lässt sich weiter vereinfachen, wenn die Schwenkachse parallel oder im Wesentlichen parallel zu der mindestens einen Zahnreihe verläuft. Im Wesentlichen parallel bedeutet insbesondere, dass die Schwenkachse parallel zu einer beispielsweise auch gekrümmten oder schwach gekrümmten Zahnreihe verlaufen kann.

Um insbesondere eine Herstellung und/oder Montage des chirurgischen Sägeblatts zu vereinfachen, ist es vorteilhaft, wenn das Verbindungsgelenk erste und zweite Verbindungselemente umfasst, die einerseits am ersten Sägeblattteil und andererseits am zweiten Sägeblattteil angeordnet oder ausgebildet sind, und wenn das erste und das zweite Verbindungselement über mindestens ein Gelenkglied miteinander gelenkig gekoppelt sind. Ein solches Verbindungsgelenk ermöglicht es, die beiden Sägeblattteile zum einen miteinander zu verbinden und zum anderen eine Beweglichkeit derselben relativ zueinander zu ermöglichen, die gewünscht ist, um auf das Sägeblatt einwirkende Kippmomente insbesondere vom ersten Sägeblattteil zu entkoppeln.

Ein besonders einfacher Aufbau des chirurgischen Sägeblatts kann insbesondere dadurch erreicht werden, dass das erste Verbindungselement in Form eines Verbindungsvorsprungs und dass das zweite Verbindungselement in Form einer Verbindungsaufnahme ausgebildet sind. Der Verbindungsvorsprung und die Verbindungsaufnahme können wahlweise am ersten und/oder am zweiten Sägeblattteil vorgesehen sein. Denkbar sind auch zwei oder mehr Verbindungselemente an jedem Sägeblattteil.

Besonders einfach und kompakt ausbilden lässt sich das chirurgische Sägeblatt, wenn das mindestens eine Gelenkglied in Form eines Lagerstifts oder einer Lagerwelle ausgebildet ist, welcher beziehungsweise welche am ersten und am zweiten Verbindungselement angeordnete oder ausgebildete Gelenkgliedaufnahmen durchsetzt. Insbesondere können die Gelenkgliedaufnahmen an den beiden Sägeblattteilen parallel zu von den beiden Sägeblattteilen jeweils definierten Ebenen verlaufen.

Vorteilhaft ist es, wenn die Gelenkgliedaufnahmen in Form von Bohrungen ausgebildet sind. Bohrungen lassen sich besonders einfach und kostengünstig herstellen.

Um eine Beschädigung der mindestens einen Zahnreihe beim Zusammenwirken des chirurgischen Sägeblatts mit einer Sägelehre nach Möglichkeit zu minimieren, ist es günstig, wenn die mindestens eine Zahnreihe an einem distalen Ende des ersten Sägeblattteils angeordnet oder ausgebildet ist.

Um eine Stabilität des chirurgischen Sägeblatts zu erhöhen, ist es günstig, wenn der erste Sägeblattteil einstückig ausgebildet ist.

Eine Stabilität des Sägeblatts lässt sich insbesondere dadurch weiter erhöhen, dass der zweite Sägeblattteil einstückig ausgebildet ist.

Die eingangs gestellte Aufgabe wird ferner bei einer chirurgischen Säge der eingangs beschrieben Art erfindungsgemäß dadurch gelöst, dass eine Oszillationsachse der Antriebseinheit senkrecht zu der die Schwenkachse enthaltenden Kupplungsebene verläuft.

Eine mit einem derart weitergebildeten chirurgischen Sägeblatt ausgestatte chirurgische Säge weist die bereits oben im Zusammenhang mit einem erfindungsgemäß vorgeschlagenen Sägeblatt dargelegten Vorteile auf. Vorteilhaft ist es, dass der erste Sägeblattteil und der zweite Sägeblattteil relativ zueinander verschwenkbar aneinander gelagert sind. Dies gestattet es, die beiden Sägeblattteile relativ zueinander in praktisch einem beliebigen Winkel zu neigen. Mit anderen Worten kann so eine geneigte Krafteinleitung auf eine chirurgische Säge durch einen Operateur ausgeglichen werden, um eine Verschiebung der Sägelehre aus der gewünschten Position zu vermeiden. Günstig ist es, dass der erste Sägeblattteil und der zweite Sägeblattteil relativ zueinander um eine Schwenkachse verschwenkbar aneinander gelagert sind. Insbesondere durch die Wahl der Schwenkachse kann eine Entkopplung zwischen dem ersten Sägeblattteil und eingeleiteten Kippmomenten sicher vermieden werden.

Gemäß einer weiteren bevorzugten Ausführungsform der Erfindung kann vorgesehen sein, dass die Antriebseinheit eine zweite Kupplungseinrichtung aufweist, welche mit der ersten Kupplungseinrichtung des Sägeblatts zum Kuppeln in Eingriff bringbar ist. Mit einer zweiten Kupplungseinrichtung an der Antriebseinheit lässt sich diese mit dem Sägeblatt auf einfache Weise verbinden, um beispielsweise Knochen mit der Säge zu bearbeiten. Ist das Sägeblatt stumpf oder verschmutzt, lässt es sich wieder auf einfache Weise von der Antriebseinheit lösen und beispielsweise durch ein neues Sägeblatt austauschen.

Vorteilhaft kann es sein, wenn die Antriebseinheit in Form eines Oszillationsantriebs ausgebildet ist zum hin und her Verschwenken der zweiten Kupplungseinrichtung um eine Oszillationsachse. Mit einer solchen Antriebseinheit kann auf einfache Weise eine Oszillationssäge ausgebildet werden.

Um eine möglichst gute Entkopplung von auf das Sägeblatt einwirkenden Kippmomenten zu erreichen, ist es günstig, wenn die Oszillationsachse quer zu einer die Schwenkachse enthaltenden Ebene verläuft. Eine solche Ausgestaltung ermöglicht eine besonders gute Kraftübertragung vom zweiten Sägeblattteil auf das erste Sägeblattteil.

Vorteilhaft ist es, wenn die chirurgische Säge eines der oben beschriebenen chirurgischen Sägeblätter umfasst. Sie weist dann auch die oben im Zusammenhang mit den bevorzugten Ausführungsformen der chirurgischen Sägeblätter beschriebenen Vorteile auf.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische schematische Darstellung einer chirurgischen Säge mit angekoppeltem chirurgischem Sägeblatt;

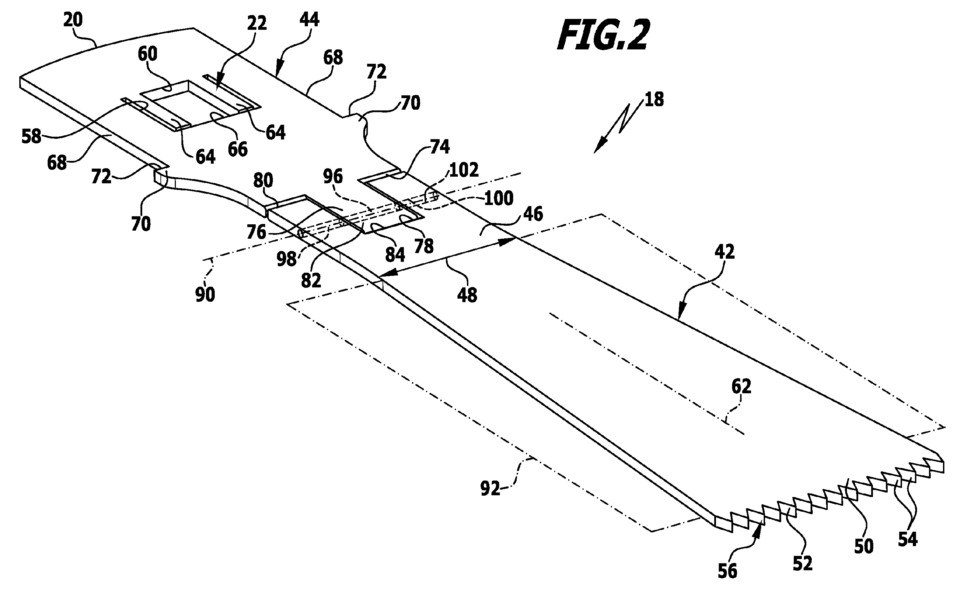

- Figur 2:: eine schematische perspektivische Ansicht des in Figur 1 dargestellten chirurgischen Sägeblatts in einer ersten Gelenkstellung; und
- Figur 3:: eine schematische perspektivische Ansicht des in Figur 2 dargestellten chirurgischen Sägeblatts in zwei weiteren Gelenkstellungen.

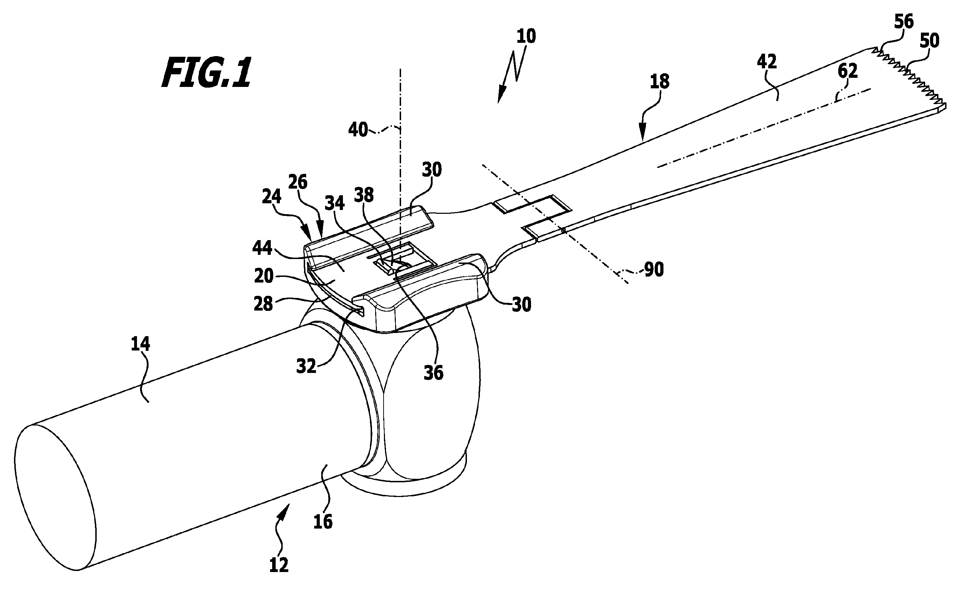

In Figur 1 ist eine insgesamt mit dem Bezugszeichen 10 bezeichnete chirurgische Säge schematisch dargestellt. Sie umfasst eine chirurgische Antriebseinheit 12, die wahlweise über ein nicht dargestelltes Verbindungskabel mit einer Netzversorgung verbindbar und somit netzstrombetrieben werden kann. Alternativ kann die Antriebseinheit 12 auch akku- oder batteriebetrieben ausgebildet sein. In diesem Fall ist dann vorzugsweise in einem langgestreckten, zylindrischen Handgriff 14 eines Gehäuses 16 der Antriebseinheit 12 eine nicht näher dargestellte Batterie untergebracht. Diese kann insbesondere auch als wiederaufladbare Batterie ausgebildet sein.

Die Säge 10 umfasst ferner ein chirurgisches Sägeblatt 18 mit einer an seinem proximalen Ende 20 beziehungsweise im Bereich seines proximalen Endes 20 ausgebildeten ersten Kupplungseinrichtung 22 zum Kuppeln beziehungsweise Verbinden des Sägeblatts 18 mit einer zweiten Kupplungseinrichtung 24 der Antriebseinheit 12.

Die zweite Kupplungseinrichtung 24 umfasst eine Halteplatte 26 mit von einer Oberseite 28 derselben abstehenden Randvorsprüngen 30, welche parallel zueinander ausgerichtet sind und aufeinander zu weisend geöffnete Längsnuten 32 umfassen. Durch eine Durchbrechung 36 in der Oberseite 28 ragt ein senkrecht zu einer von der Oberseite 28 definierten Ebene beweglich, vorzugsweise verschiebbarer, Verriegelungszapfen 34, mit einer in distaler Richtung abgeschrägten Aufgleitfläche 38 heraus.

Die Halteplatte 26 ist mit einem im Gehäuse 16 angeordneten Antrieb gekoppelt, vorzugsweise einem elektromotorischen Antrieb, welcher eine senkrecht zur Oberseite 28 verlaufende Oszillationsachse 40 definiert, relativ zu welcher die Halteplatte 26, angetrieben durch den Antrieb, hin und her schwingen beziehungsweise oszillieren kann. Die Antriebseinheit 12 ist somit in Form eines Oszillationsantriebs ausgebildet zum hin und her Verschwenken der zweiten Kupplungseinrichtung 24 um die Oszillationsachse 40.

Das Sägeblatt 18 umfasst einen ersten Sägeblattteil 42 und einen zweiten Sägeblattteil 44. Der erste Sägeblattteil 42 weist einen proximalen Endbereich 46 auf, welcher eine minimale Breite 48 des Sägeblatts 18 definiert. Ausgehend vom Endbereich 46, welcher sich etwa über ein Fünftel der Länge des ersten Sägeblattteils 42 erstreckt, nimmt die Breite des ersten Sägeblattteils 42 in distaler Richtung bis zu ihrem distalen Ende 50 leicht zu. Eine in distaler Richtung weisende Stirnkante 52 des ersten Sägeblattteils 42 ist mit einer Mehrzahl von Zähnen 54 zur Ausbildung einer Zahnreihe 56 versehen. Die Zähne 54 weisen in distaler Richtung.

Der zweite Sägeblattteil 44 umfasst die erste Kupplungseinrichtung 22. Diese umfasst zum einen eine rechteckige Durchbrechung 58, von deren in distaler Richtung weisender Innenkante 60 zwei in Längsrichtung parallel zu einer vom Sägeblatt 18 definierten Längsachse 62 in distaler Richtung weisende quaderförmige Vorsprünge 64 abstehen, die bis fast zu einer der Innenkante 60 gegenüberliegenden Innenkante 66 heranreichen. Die erste Kupplungseinrichtung 22 umfasst zum anderen zwei symmetrisch zu einer die Längsachse 62 enthaltenden Spiegelebene ausgebildete und in entgegengesetzte Richtungen weisend von Seitenkanten 68 abstehende Anschlägen 70, welche Seitenkanten ebenfalls in entgegengesetzte Richtungen weisend ausgebildet sind. Die Anschläge 70 definieren in proximaler Richtung weisende und eine gemeinsame, senkrecht zur Längsachse 62 verlaufende Ebene definierende Anschlagflächen 72.

Von einer in distaler Richtung weisenden Stirnkante 74 des zweiten Sägeblattteils 44 steht ein in distaler Richtung weisender Verbindungsvorsprung 76 ab, welcher in eine korrespondierende Verbindungsaufnahme 78 des ersten Sägeblattteils 42 im Wesentlichen formschlüssig eingreift. Die Verbindungsaufnahme 78 erstreckt sich ausgehend von einer in proximaler Richtung weisenden Stirnkante 80 des ersten Sägeblattteils 42 in distaler Richtung. Der Verbindungsvorsprung 76 und die Verbindungsaufnahme 78 bilden erste und zweite Verbindungselemente 82 und 84 eines insgesamt mit dem Bezugszeichen 86 bezeichneten Verbindungsgelenks zum gelenkigen Verbinden der beiden Sägeblattteile 42, 44 miteinander.

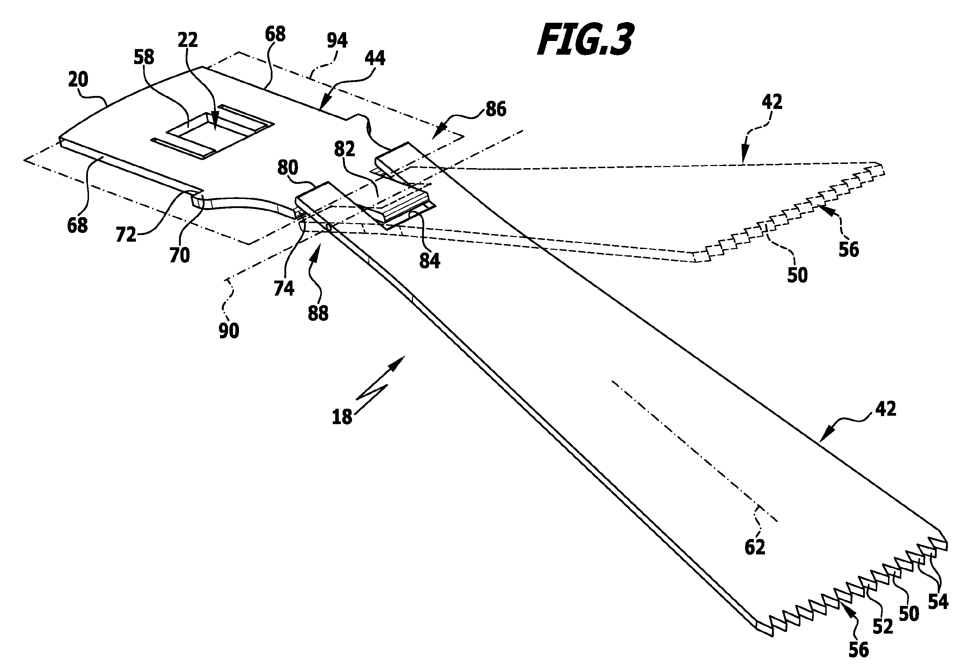

Das Verbindungsgelenk 86 ist vorzugsweise in Form eines Scharniergelenks 86 ausgebildet und gestattet so eine Verschwenkung der Sägeblattteile 42 und 44 relativ zueinander um eine Schwenkachse 90. Die Schwenkachse 90 liegt in einer vom ersten Sägeblattteil 42 definierten Sägeebene 92. Ebenso liegt die Schwenkachse 90 in einer vom zweiten Sägeblattteil 42 definierten Kupplungsebene 94. Die Kupplungsebene 94 verläuft zudem senkrecht zur Oszillationsachse 40, wenn das Sägeblatt 18 mit der Antriebseinheit 12 gekoppelt beziehungsweise verbunden ist. Die Schwenkachse 90 wird definiert durch eine Längsachse eines Gelenkglieds 96 in Form eines langgestreckten, zylindrischen, eine Lagerwelle bildenden Lagerstifts 98, welcher in einer Bohrung 100, die Gelenkgliedaufnahmen 102 in beiden Verbindungselementen 82, 84 quer zur Längsachse 62 definiert, durchsetzt. Die den Verbindungsvorsprung 76 durchsetzende Bohrung 100 ist im Innendurchmesser ein wenig größer bemessen als ein Außendurchmesser des Lagerstifts 98. Der Lagerstift 98 ist in der Bohrung 100 am ersten Sägeblattteil 42 unbeweglich festlegt. Dies bedeutet, dass der Verbindungsvorsprung 76 um den Lagenstift 98 verschwenkt werden kann, wie dies schematisch in Figur 3 dargestellt ist.

Zum Verbinden des Sägeblatts 18 wird der zweite Sägeblattteil 44 mit seinem Ende 20 an die zweite Kupplungseinrichtung 24 heran und seitlich in die aufeinander zu weisenden Längsnuten 32 eingeführt. Sobald das Ende 20 den Verriegelungszapfen 34 erreicht, gleitet das Ende 20 an der Aufgleitfläche 38 auf und drückt den Verriegelungszapfen 34 entgegen der Wirkung einer nicht dargestellten Feder vom Sägeblatt 18 weg in Richtung auf das Gehäuse 16 hin. Sobald das Sägeblatt 18 so weit in proximaler Richtung vorgeschoben ist, dass der Verriegelungszapfen 34 zwischen den Vorsprüngen 38 wieder in die Durchbrechung 58 eingreifen kann, schnappt er in seine in Figur 1 dargestellte Ausgangs- oder Kupplungsstellung zurück und liegt mit einer in proximaler Richtung weisenden Kante an der Innenkante 60 an. Auf diese Weise wird eine Relativbewegung des Sägeblatts 18 zur zweiten Kupplungseinrichtung 24 in distaler Richtung verhindert. Die Anschlagflächen 72 liegen in dieser Stellung an in distaler Richtung weisenden Stirnflächen der Randvorsprünge 60 an.

Zum Bearbeiten von Knochen kann nun der erste Sägeblattteil 42 mit der Zahnreihe 56 voran durch einen Sägeschlitz einer dafür vorgesehenen Sägelehre, beispielsweise in Form einer Sägeschablone, hindurchgeführt werden. Durch Vorsehen des Verbindungsgelenks 86 ist praktisch eine verlustlose Kraftübertragung in der Sägeebene 92 möglich, da eine vollständige Entkopplung des Kraftschlusses senkrecht zur Sägeebene 92 erreicht wird.

Beide Sägeblattteile 42 und 44 können insbesondere aus einem flachen Metallstreifen hergestellt und vorzugsweise jeweils einstückig ausgebildet werden. Sie weisen beide günstigerweise dieselbe Dicke auf.

## Patentansprüche

1. Chirurgisches Sägeblatt (18) mit mindestens einer Zahnreihe (56) und einer Kupplungseinrichtung (22) zum beweglichen Kuppeln und/oder Verbinden des Sägeblatts (18) mit einer chirurgischen, eine Oszillationsachse (40) definierenden Antriebseinheit (12), wobei das Sägeblatt (18) einen ersten und einen zweiten Sägeblattteil (42, 44) umfasst, wobei der erste Sägeblattteil (42) die mindestens eine Zahnreihe (56) umfasst, wobei der zweite Sägeblattteil (44) die Kupplungseinrichtung (22) umfasst, wobei der erste Sägeblattteil (42) und der zweite Sägeblattteil (44) gelenkig miteinander verbunden sind, wobei der erste Sägeblattteil (42) und der zweite Sägeblattteil (44) relativ zueinander um eine Schwenkachse (90) verschwenkbar aneinander gelagert sind, wobei der zweite Sägeblattteil (44) eine Kupplungsebene (94) definiert und wobei die Kupplungsebene (94) die Schwenkachse (90) enthält, **dadurch gekennzeichnet, dass** die Oszillationsachse (40) senkrecht zu der die Schwenkachse (90) enthaltenden Kupplungsebene (94) verläuft.

2. Chirurgisches Sägeblatt nach Anspruch 1, **gekennzeichnet durch** ein Verbindungsgelenk (86) zum gelenkigen Verbinden des ersten Sägeblattteils (42) und des zweiten Sägeblattteils (44) miteinander.

3. Chirurgisches Sägeblatt nach Anspruch 2, **dadurch gekennzeichnet, dass** das Verbindungsgelenk (86) in Form eines Scharniergelenks (88) ausgebildet ist.

4. Chirurgisches Sägeblatt nach einem der voranstehenden Ansprüche, **dadurch gekennzeichnet, dass** der erste Sägeblattteil (42) eine Sägeebene (92) definiert und dass die Sägeebene (92) die Schwenkachse (90) enthält.

5. Chirurgisches Sägeblatt nach einem der voranstehenden Ansprüche, **dadurch gekennzeichnet, dass** die Schwenkachse (90) parallel oder im Wesentlichen parallel zu der mindestens einen Zahnreihe (56) verläuft.

6. Chirurgisches Sägeblatt nach einem der Ansprüche 2 bis 5, **dadurch gekennzeichnet, dass** das Verbindungsgelenk (86) erste und zweite Verbindungselemente (82, 84) umfasst, die einerseits am ersten Sägeblattteil (42) und andererseits am zweiten Sägeblattteil (44) angeordnet oder ausgebildet sind, und dass das erste und das zweite Verbindungselement (82, 84) über mindestens ein Gelenkglied (96) miteinander gelenkig gekoppelt sind.

7. Chirurgisches Sägeblatt nach Anspruch 6, **dadurch gekennzeichnet, dass** das erste Verbindungselement (82) in Form eines Verbindungsvorsprungs (76) und dass das zweite Verbindungselement (84) in Form einer Verbindungsaufnahme (78) ausgebildet sind.

8. Chirurgisches Sägeblatt nach Anspruch 6 oder 7, **dadurch gekennzeichnet, dass** das mindestens eine Gelenkglied (96) in Form eines Lagerstifts (98) oder einer Lagerwelle ausgebildet ist, welcher beziehungsweise welche am ersten und am zweiten Verbindungselement (82, 84) angeordnete oder ausgebildete Gelenkgliedaufnahmen (102) durchsetzt.

9. Chirurgisches Sägeblatt nach einem der voranstehenden Ansprüche, **dadurch gekennzeichnet, dass** die mindestens eine Zahnreihe (56) an einem distalen Ende des ersten Sägeblattteils (42) angeordnet oder ausgebildet ist.

10. Chirurgisches Sägeblatt nach einem der voranstehenden Ansprüche, **dadurch gekennzeichnet, dass** der erste Sägeblattteil (42) einstückig ausgebildet ist.

11. Chirurgisches Sägeblatt nach einem der voranstehenden Ansprüche, **dadurch gekennzeichnet, dass** der zweite Sägeblattteil (44) einstückig ausgebildet ist.

12. Chirurgische Säge (10) umfassend eine chirurgische, eine Oszillationsachse definierende Antriebseinheit (12) und ein mit der Antriebseinheit (12) lösbar verbindbares chirurgisches Sägeblatt (18) gemäß Anspruch 1.

13. Chirurgische Säge nach Anspruch 12, **dadurch gekennzeichnet, dass** die Kupplungseinrichtung (22) eine erste Kupplungseinrichtung (22) ist und dass die Antriebseinheit (12) eine zweite Kupplungseinrichtung (24) aufweist, welche mit der ersten Kupplungseinrichtung (22) des Sägeblatts (18) zum Kuppeln in Eingriff bringbar ist.

14. Chirurgische Säge nach Anspruch 13, **dadurch gekennzeichnet, dass** die Antriebseinheit (12) in Form eines Oszillationsantriebs ausgebildet ist zum hin und her Verschwenken der zweiten Kupplungseinrichtung (24) um eine Oszillationsachse (40).

15. Chirurgische Säge nach einem der Ansprüche 12 bis 14, **gekennzeichnet durch** ein Sägeblatt (18) nach einem der Ansprüche 2 bis 11.

## Claims

1. Surgical saw blade (18) having at least one row of teeth (56) and a coupling device (22) for movably coupling and/or connecting the saw blade (18) to a surgical drive unit (12) defining an oscillation axis (40), wherein the saw blade (18) comprises a first and a second saw blade part (42, 44), wherein the first saw blade part (42) comprises the at least one row of teeth (56), wherein the second saw blade part (44) comprises the coupling device (22), wherein the first saw blade part (42) and the second saw blade part (44) are articulately connected together, wherein the first saw blade part (42) and the second saw blade part (44) are supported on each other for pivotal movement relative to each other about a pivot axis (90), wherein the second saw blade part (44) defines a coupling plane (94) and wherein the coupling plane (94) contains the pivot axis (90), **characterized in that** the oscillation axis (40) extends perpendicularly to the coupling plane (94) containing the pivot axis (90).

2. Surgical saw blade in accordance with claim 1, **characterized by** an articulated connecting joint (86) for articulately connecting together the first saw blade part (42) and the second saw blade part (44).

3. Surgical saw blade in accordance with claim 2, **characterized in that** the articulated connecting joint (86) is configured in the form of an articulated hinged joint (88).

4. Surgical saw blade in accordance with any one of the preceding claims, **characterized in that** the first saw blade part (42) defines a sawing plane (92) and **in that** the sawing plane (92) contains the pivot axis (90).

5. Surgical saw blade in accordance with any one of the preceding claims, **characterized in that** the pivot axis (90) is parallel or essentially parallel to the at least one row of teeth (56).

6. Surgical saw blade in accordance with any one of claims 2 to 5, **characterized in that** the articulated connecting joint (86) comprises first and second connecting elements (82, 84) which are arranged or formed, on the one hand, on the first saw blade part (42) and, on the other hand, on the second saw blade part (44) and **in that** the first and the second connecting element (82, 84) are articulately coupled together via at least one articulation member (96).

7. Surgical saw blade in accordance with claim 6, **characterized in that** the first connecting element (82) is configured in the form of a connecting projection (76) and **in that** the second connecting element (84) is configured in the form of a connecting receptacle (78).

8. Surgical saw blade in accordance with claim 6 or 7, **characterized in that** the at least one articulation member (96) is configured in the form of a bearing pin (98) or a bearing shaft which extends through articulation member receptacles (102) arranged at the first and at the second connecting element (92, 84).

9. Surgical saw blade in accordance with any one of the preceding claims, **characterized in that** the at least one row of teeth (56) is arranged or formed at a distal end of the first saw blade part (42).

10. Surgical saw blade in accordance with any one of the preceding claims, **characterized in that** the first saw blade part (42) is configured in one piece.

11. Surgical saw blade in accordance with any one of the preceding claims, **characterized in that** the second saw blade part (44) is configured in one piece.

12. Surgical saw (10), comprising a surgical drive unit (12) defining an oscillation axis and a surgical saw blade (18) releasably connectable to the drive unit (12) in accordance with claim 1.

13. Surgical saw in accordance with claim 12, **characterized in that** the coupling device (22) is a first coupling device (22) and **in that** the drive unit (12) comprises a second coupling device (24) engageable with the first coupling device (22) of the saw blade (18) for coupling therewith.

14. Surgical saw in accordance with claim 13, **characterized in that** the drive unit (12) is configured in the form of an oscillatory drive for pivoting the second coupling device (24) back and forth about an axis of oscillation (40).

15. Surgical saw in accordance with any one of claims 12 to 14, **characterized by** a saw blade (18) in accordance with any one of claims 2 to 11.

## Revendications

1. Lame de scie chirurgicale (18) comprenant au moins une rangée de dents (56) et un dispositif d'accouplement (22) pour coupler et/ou relier de manière mobile la lame de scie (18) à une unité d'entraînement chirurgicale (12) définissant un axe d'oscillation (40),
la lame de scie (18) comprenant une première et une deuxième partie de lame de scie (42, 44),
la première partie de lame de scie (42) comprenant ladite au moins une rangée de dents (56),
la deuxième partie de lame de scie (44) comprenant le dispositif d'accouplement (22),
la première partie de lame de scie (42) et la deuxième partie de lame de scie (44) étant reliées de manière articulée l'une à l'autre,
la première partie de lame de scie (42) et la deuxième partie de lame de scie (44) étant montées l'une sur l'autre en pouvant pivoter l'une par rapport à l'autre autour d'un axe de pivotement (90),
la deuxième partie de lame de scie (44) définissant un plan de couplage (94),
et le plan de couplage (94) renfermant l'axe de pivotement (90),
**caractérisée en ce que** l'axe d'oscillation (40) s'étend perpendiculairement au plan de couplage (94) renfermant l'axe de pivotement (90).

2. Lame de scie chirurgicale selon la revendication 1,
**caractérisée par** une articulation de liaison (86) pour assurer la liaison articulée de la première partie de lame de scie (42) et de la deuxième partie de lame de scie (44) l'une avec l'autre.

3. Lame de scie chirurgicale selon la revendication 2,
**caractérisée en ce que** l'articulation de liaison (86) est réalisée sous la forme d'une articulation à charnière (88).

4. Lame de scie chirurgicale selon l'une des revendications précédentes, **caractérisée en ce que** la première partie de lame de scie (42) définit un plan de sciage (92) et **en ce que** le plan de sciage (92) contient l'axe de pivotement (90).

5. Lame de scie chirurgicale selon l'une des revendications précédentes, **caractérisée en ce que** l'axe de pivotement (90) s'étend parallèlement ou sensiblement de manière parallèle à ladite au moins une rangée de dents (56).

6. Lame de scie chirurgicale selon l'une des revendications 2 à 5, **caractérisée en ce que** l'articulation de liaison (86) comprend des premiers et deuxièmes éléments de liaison (82, 84), qui sont agencés ou formés d'une part sur la première partie de lame de scie (42) et d'autre part sur la deuxième partie de lame de scie (44), et **en ce que** le premier et le deuxième élément de liaison (82, 84) sont couplés mutuellement de manière articulée, par l'intermédiaire d'au moins un organe d'articulation (96).

7. Lame de scie chirurgicale selon la revendication 6,
**caractérisée en ce que** le premier élément de liaison (82) est réalisé sous la forme d'une protubérance de liaison (76), et **en ce que** le deuxième élément de liaison (84) est réalisé sous la forme d'un logement d'accueil de liaison (78).

8. Lame de scie chirurgicale selon la revendication 6 ou la revendication 7, **caractérisée en ce que** ledit au moins un organe d'articulation (96) est réalisé sous la forme d'une goupille de palier (98) ou d'un arbre de palier, qui traverse des logements d'accueil d'organe d'articulation (102) agencés ou formés sur le premier et le deuxième élément d'articulation (82, 84).

9. Lame de scie chirurgicale selon l'une des revendications précédentes, **caractérisée en ce que** ladite au moins une rangée de dents (56) est agencée ou formée à une extrémité distale de la première partie de lame de scie (42).

10. Lame de scie chirurgicale selon l'une des revendications précédentes, **caractérisée en ce que** la première partie de lame de scie (42) est réalisée d'un seul tenant.

11. Lame de scie chirurgicale selon l'une des revendications précédentes, **caractérisée en ce que** la deuxième partie de lame de scie (44) est réalisée d'un seul tenant.

12. Scie chirurgicale (10) comprenant une unité d'entraînement chirurgicale (12) définissant un axe d'oscillation, et une lame de scie (18) selon la revendication 1, qui peut être reliée de manière amovible à l'unité d'entraînement (12).

13. Scie chirurgicale selon la revendication 12,
**caractérisée en ce que** le dispositif d'accouplement (22) est un premier dispositif d'accouplement (22), et **en ce que** l'unité d'entraînement (12) présente un deuxième dispositif d'accouplement (24), qui peut être amené en prise avec le premier dispositif d'accouplement (22) de la lame de scie (18), pour le couplage.

14. Scie chirurgicale selon la revendication 13,
**caractérisée en ce que** l'unité d'entraînement (12) est réalisée sous la forme d'un entraînement oscillant pour faire pivoter en va et vient le deuxième élément d'accouplement (24) autour d'un axe d'oscillation (40).

15. Scie chirurgicale selon l'une des revendications 12 à 14, **caractérisée par** une lame de scie (18) selon l'une des revendications 2 à 11.
